# EUROPEAN PATENT APPLICATION

(11) **EP 1 715 033 A1**
(43) Date of publication of application: **25.10.2006**
(21) Application number: 05710079.4
(22) Date of filing: 10.02.2005
(51) Int. Cl.: C12N 5/06, A61K 35/36, A61K 48/00, A61L 27/00

(54) **MEDIUM FOR PREPARING FEEDER CELLS FOR EMBRYONIC STEM CELLS AND FEEDER CELLS**

(30) Priority: 13.02.2004 JP 2004036845
(71) Applicant: ReproCELL Inc., Tokyo 100-0011 (JP); ASAHI TECHNO GLASS COSPORATION, Chiba 273-0044 (JP); TANABE SEIYAKU CO., LTD., Chuo-ku, Osaka-shi, Osaka 541-8505 (JP)
(72) Inventor: NAKATSUJI, Norio, Kyoto-shi, Kyoto 6060014 (JP); SUEMORI, Hirofumi, Sakyo-ku, Kyoto-shi, Kyoto 6068397 (JP); ASAKA, Isao, Asahi Techno Glass Corporation, Funabashi-shi, Chiba 2730044 (JP); SUGAI, Harumi, Asahi Techno Glass Corporation, Funabashi-shi, Chiba 2730044 (JP); OKAMOTO, Reiko, Asahi Techno Glass Corporation, Funabashi-shi, Chiba 2730044 (JP)
(74) Representative: Zwicker, Jörk
(86) International application number: PCT/JP2005/002027
(87) International publication number: WO 2005/078070

(57) **Abstract**

A culture medium for preparation of feeder cells for embryonic stem cells, which can efficiently establish feeder cells for use in culture of embryonic stem cells including human's from limited donor-derived materials and culture them in a condition of a reduced risk of infection, is provided. Further, a preparation method of feeder cells, which is relatively safe even when subjected to coculture with embryonic stem cells including human's, and the resulting feeder cells therefrom are provided. With the culture medium for preparation of feeder cells for embryonic stem cells comprising at least a serum albumin and insulin in a basal medium, a cell population comprising at least one kind of cells selected from fetal skin fibroblasts, fetal myofibroblasts, fetal lung fibroblasts, fetal epithelial cells, fetal endothelial cells, adult skin fibroblasts, adult lung fibroblasts, adult epithelial cells and endothelial cells which can become feeder cells for embryonic stem cells can be stably proliferated.

## Description

### Technical Field

The present invention relates to a culture medium for preparation of feeder cells for use in culturing embryonic stem cells including human's, the preparation method of feeder cells employing the culture medium, and the resulting feeder cells therefrom for embryonic stem cells.
This application claims the priority of Japanese Patent Application No.2004-36845, which is incorporated herein by reference.

### Background Art

Embryonic stem cells are undifferentiated cells derived from the inner cell mass of a blastocyst and have a pluripotency to differentiate into all tissue types; thereby its application is expected in the fields of cell culturing, tissue transplantation, drug discovery research, and gene therapy. Further, it is known that embryonic stem cells may be induced from a cloned embryo obtained by the nuclear transfer from adult somatic cells. Recently, technologies inducing embryonic stem cells to be developed into various tissues including nerve tissues have been reported, and as tissues developed from embryonic stem cells are genetically equivalent tissues without immunologically being eliminated, so that their application to transplantation and gene therapies has become expected. Further, subjects for which embryonic stem cells are produced have become diverse covering from experimental small animals to primates including human, and the isolation of embryonic stem cells of rhesus monkey, the separation of that from a human ovum fertilized in vitro and the like have been reported.

Further, Suemori et al. succeeded in newly develop to establish embryonic stem cells by intracytoplasmic sperm injection from a cynomolgus monkey (Macaca fascicularis) which is useful for preclinical studies and widely employed in medical researches, and shown its pluripotency maintained over a long period, and also confirmed that dopaminergic neurons are generated from the embryonic stem cells of a Cynomolgus monkey.

Embryonic stem cells including human's described above have conventionally been cocultured with feeder cells to facilitate their growth. When culture of embryonic stem cells of a primate including, in particular, human using a serum, an undifferentiated state of them cannot be maintained, so that culture them with a serum-free medium has been studied (see patent document No.1).

However, embryonic stem cells cannot be maintained when culture only with a serum-free medium, therefore feeder cells, which partly substitute for the effects of a serum, are essential for the coculture. As the feeder cells, for example, cells obtained by preparing fibroblasts from a fetal mouse and subjecting them to mitomycin C or irradiation to inactivate their proliferation have been used, so that coculture human embryonic stem cells on feeder layer derived from a mouse could lead to zoonosis and the like. As this tissue induced and developed from human embryonic stem cells is expected to be as a material for transplantation and gene therapies, risks like zoonosis should desirably be eliminated as much as possible.

Thus, instead of using a fetal mouse, methods using human fetal fibroblasts, adult oviduct epithelial cells (non-patent document No.1), human neonatal foreskin-derived fibroblasts (non-patent document No.2, 3), adult skin fibroblasts (non-patent document No.4) and human bone marrow cells (non-patent document No.5) as feeder cells have been reported.

However, most of them use primary cells, thereby to obtain a large amount of human embryonic stem cells; materials derived from a plurality of donors are needed, so that checking on every donor for the contagium is also laboriously needed. In addition, these culture media for preparation of feeder cells derived from a human use a serum such as fetal bovine serum (FBS), leading a risk of spread of unknown pathogens such as unknown infections and prion from this serum.
[Patent document No.1] Pamphlet of International Publication No.98/30679
[Non-patent document No.1] Nat. Biotechnol.20:933-936 (2002)
[Non-patent document No.2] Biol. Reprod.68:2150-2156 (2003)
[Non-patent document No.3] Hum. Reprod.18:1404-1409 (2003)
[Non-patent document No.4] Stem Cells.21:546-556 (2003)
[Non-patent document No.5] Stem Cells.21:131-142 (2003)

### Disclosure of the Invention

### (Problems to be Solved by the Invention)

The object of the present invention is to provide a culture medium for preparation of feeder cells for embryonic stem cells, which can be used to efficiently establish feeder cells for use in culture of embryonic stem cells including human's from limited donor-derived materials and to culture them in a condition of a reduced risk of infection (hereinafter referred to as "a culture medium for preparation of feeder cells"). Also, a preparation method of feeder cells, which is relatively safe even when subjected to coculture with embryonic stem cells, and the resulting feeder cells there from are provided.

### (Means to Solve the Problems)

The present inventors had studied to solve the matters above and found that using a culture medium for preparation of feeder cells, comprising at least a serum albumin and insulin in a basal medium, a cell population capable of becoming feeder cells for embryonic stem cells and comprising at least one kind of cells selected from fetal skin fibroblasts, fetal fibroblasts, fetal lung fibroblasts, fetal epithelial and endothelial cells, adult skin and lung fibroblasts, adult epithelial and endothelial cells can be stably proliferated, and they completed the present invention.

Therefore, the present invention consists of the following:
1. A culture medium for preparation of feeder cells for embryonic stem cells, comprising serum albumin, insulin and a basal medium, wherein the amount of the serum albumin is from 2 g/L to 50 g/L, the amount of the insulin is from 1 mg/L to 100 mg/L, and the basal medium is at least one kind selected from MEM, α-MEM, DMEM, IMDM, Ham F10, Ham F12, Medium 199, RPMI 1640, RITC 80-7, MCDB 104, MCDB 105, MCDB 153, MCDB 201 and MCDB 202.
2. The culture medium according to preceding aspect 1 further comprising a cell adhesion factor.
3. The culture medium according to preceding aspect 2, wherein the cell adhesion factor is at least one kind selected from collagen, gelatin, fibronectin, vitronectin, laminin, polylysine, polyornithine and polyethyleneimine.
4. The culture medium according to preceding aspects 1, 2 or 3 further comprising a cell growth factor.
5. The culture medium according to preceding aspect 4, wherein the cell growth factor is at least one kind selected from fibroblast growth factor and epithelial cell growth factor.
6. A method for preparation of feeder cells for embryonic stem cells comprising the steps of:
   culture and proliferation a cell population comprising at least one kind of cells selected from fetal skin fibroblasts, fetal myofibroblasts, fetal lung fibroblasts, fetal epithelial cells, fetal endothelial cells, adult skin fibroblasts, adult lung fibroblasts, adult epithelial cells and adult endothelial cells in the culture medium for preparation of feeder cells for embryonic stem cells according to any one of preceding aspects 1 through 5, and
   inactivation of proliferation of the cultured and proliferated cell population by mitomycin C or irradiation.
7. The preparation method according to preceding aspect 6, wherein the culture and proliferation step is conducted in a culture vessel coated with a cell adhesion factor.
8. The preparation method according to preceding aspect 7, wherein the cell adhesion factor is at least one kind selected from collagen, gelatin, fibronectin, vitronectin, laminin, polylysine, polyornithine and polyethyleneimine.
9. The preparation method according to preceding aspects 6, 7 or 8, wherein in the culture and proliferation step, the cultured cells are allowed to undergo cell division twenty or more times on average.
10. Feeder cells for embryonic stem cells obtained by the preparation method according to any one of preceding aspects 6 through 9.

### (Effects of Invention)

With a culture medium for preparation of feeder cells of the present invention, feeder cells which are relatively safe even subjected to coculture with embryonic stem cells including human's can be established and cultured over a long period. Materials for feeder cells are derived from limited donors, for example, fetal skin fibroblasts, fetal myofibroblasts, fetal lung fibroblasts, fetal epithelial cells, fetal endothelial cells, adult skin fibroblasts, adult lung fibroblasts, adult epithelial cells and adult endothelial cells and the like, therefore capability of a long-term culture is useful. With it, coculture of embryonic stem cells including human's with feeder cells for embryonic stem cells can be achieved, providing embryonic stem cells which have a reduced risk of zoonosis and the like in case of using embryonic stem cells as a material for transplantation and gene therapies.

### Brief Description of the Drawings

Fig. 1 shows a photo of 10 day-old colony (x 40) of embryonic stem cells from a cynomolgus monkey, which were cultured on feeder layer prepared in Example 2.
Fig. 2 shows a photo of 4 day-old colony (x 100) of embryonic stem cells from a cynomolgus monkey after subcultured, which were cultured on feeder layer prepared in Example 2.
Fig. 3 is a figure showing the proliferation of embryonic stem cells from a cynomolgus monkey, which were cultured on feeder layer prepared in Example 2.
Fig. 4 shows an immunostained image (x 100) with SSEA-4 of 4 day-old colony of embryonic stem cells from a cynomolgus monkey after subcultured, which were cultured on feeder layer prepared in Example 2.
Fig. 5 shows a photo of 10 day-old colony (x 40) of embryonic stem cells from a cynomolgus monkey, which were cultured on feeder layer prepared in Example 3.
Fig. 6 shows a photo of 4 day-old colony (x 100) of embryonic stem cells from a cynomolgus s monkey after subcultured, which were cultured on feeder layer prepared in Example 3.
Fig. 7 is a figure showing the proliferation of embryonic stem cells from a cynomolgus monkey, which were cultured on feeder layer prepared in Example 3.
Fig. 8 is a figure showing the proliferation of MRC-5 cultured using a gelatin coat in combination with a culture medium for preparation of feeder cells (B) in Example 4. The starting PDL of culture and the ending one which was calculated by counting cells at following subculture, were illustrated.
Fig. 9 is a figure showing the proliferation of MRC-5 cultured using a collagen coat in combination with a culture medium for preparation of feeder cells (B) in Example 5. The starting PDL of culture and the ending one which was calculated by counting cells at following subculture, were illustrated.

### Description of the Preferred Embodiment

As a basal medium used in the present invention, any one of and/or a combination of a plurality of MEM, α-MEM, DMEM, IMDM, Ham F10, Ham F12, Medium 199, RPMI 1640, RITC 80-7, MCDB 104, MCDB 105, MCDB 153, MCDB 201 and MCDB 202 can be used, preferably DMEM, IMDM, Ham F10, Ham F12, RITC 80-7, MCDB 104, MCDB 105, MCDB 201 and MCDB 202, which may be used for a serum-free medium for fibroblasts, and more preferably a formulated culture medium of DMEM and Ham F12 in 1:1, which may be used in a serum-free medium for embryonic stem cells from a primate, is used in view of nutritive value of a culture medium and the survival rate when coculture with embryonic stem cells after preparation of feeder cells.
Then, the detailed composition of a basal medium used in the present invention is based on sources described in Table 1.
[Table 1]

**Table 1 Sources of basal media**

| basal media | Sources |
|---|---|
| **MEM, a-MEM, DMEM, IMDM, RPMI1640** | The Japanese Tissue Culture Association Ed. "Tissue Culture Technology", 3rd ed. (Advanced) 1996, p 581-583 |
| **Medium 199, MCDE 104, MCDB 153** | The Japanese Tissue Culture Association Ed. "Tissue Culture Technology" in The 18th series of New Experimental Chemistry, 1990, p 27-29 |
| **Ham F10, Ham F12, MCDB 105, MCDB 202** | Katsuta H. ed. "Nutritional Requirements of Cultured Cells" 1978 63-115 |
| **MCDB 201** | Mckeehan, W. L. and Ham. R. G. 1976 J. Cell Biol 71 727-734 |
| **RITC 80-7** | Yemene, I. et al. 1981 Exp. Cell Res. 134 470-474 |

A culture medium for preparation of feeder cells used in the present invention comprising serum albumin and insulin as essential ingredients. This culture medium can further contain other ingredients. However, ingredients contain unknown ingredients such as fetal bovine serum (FBS) and contaminants and having a high possibility of causing infection and the like are inadequate.

As other ingredients, there are ingredients not contained in the above-described basal medium, or ingredients though contained there but the amount is not enough. In particular, for example, cell adhesion factors, cell growth factors, metal-containing proteins such as transferrin, other polypeptides and proteins, amino acids, vitamins may be mentioned. These ingredients are formulated into a basal medium to produce a culture medium for preparation of feeder cells. Further, such ingredients called serum replacements, which are commercially available, can be formulated into the basal medium described above to produce a culture medium for preparation of feeder cells. These serum replacements usually contain serum albumin and insulin, and further contain the other ingredients described above. Therefore, (a) serum replacement(s) can be used by formulating into the basal medium such that the amounts of serum albumin and insulin are to be the aimed amounts. As (a) serum replacement (s), for example, ones described in above patent document No.1 may be mentioned.

A culture medium for preparation of feeder cells in the present invention preferably comprising a cell adhesion factor as the other ingredient described above. In addition, a cell growth factor is preferably comprised. Further, a metal-containing protein such as transferrin is also preferably comprised. A cell adhesion factor is preferably at least one kind selected from collagen, gelatin, fibronectin, vitronectin, laminin, polylysine, polyornithine and polyethyleneimine, and in particular, collagen, gelatin, fibronectin and vitronectin are preferred. A cell growth factor is preferably at least one kind selected from fibroblast growth factor (FGF) and epithelial cell growth factor (EGF).

A culture medium for preparation of feeder cells in the present invention essentially comprising serum albumin in a ratio of from 2 g/L to 50 g/L and insulin from 1 mg/L to 100 mg/L. More preferred amounts are from 4 g/L to 25 g/L for serum albumin and from 5 mg/L to 30 mg/L for insulin.

Further, a cell adhesion factor is preferably contained at a concentration from 0.3 mg/L to 50 mg/L. However, a culture medium for preparation of feeder cells need not be formulated with a cell adhesion factor when that factor is used to coat the inner surface of a culture vessel as described below. Preferably, a cell growth factor is contained in a culture medium for preparation of feeder cells in a ratio of from 0.01 µg/L to 100 µg/L, and in particular, preferably a fibroblast growth factor from 0.1 µg/L to 10 µg/L, and more preferably an epithelial cell growth factor from 0.5 µg/L to 50 µg/L. A metal-containing protein such as transferrin is preferably contained in a ratio of from 1 mg/L to 50 mg/L.

As cells available for establishing feeder cells for embryonic stem cells of the present invention, for example, at least one kind of cells from fetal skin fibroblasts, fetal myofibroblasts, fetal lung fibroblasts, fetal epithelial cells, fetal endothelial cells, adult skin fibroblasts, adult lung fibroblasts, adult epithelial cells and adult endothelial cells can be selected. A cell population comprising a selected cell species can be cultured and grown in a culture vessel containing a culture medium for preparation of feeder cells of the present invention under a condition in 3% to 10% CO₂ at a temperature of 35°C to 40°C for 1 to 30 days. After growing the cell population described above, cell proliferation can be inactivated by mitomycin C or irradiation to obtain a large amount of feeder cells.

As stated above, safe feeder cells for embryonic stem cells, which are almost free of zoonosis, can be grown under a serum-free culture and prepared by a preparation method comprising the steps of proliferation cells and inactivation of the proliferation. In the cell proliferation step described above, a culture vessel for cells can be coated with a cell adhesion factor beforehand. In the cell proliferation step, the cultured cells are allowed to undergo cell division twenty or more times on average, obtaining a large amount of feeder cells.

### (Example)

Examples and Comparative Examples of the present invention will be explained below but these Examples show just one implementation aspect for the purpose of assisting the reproduction of the present invention, so these Examples will never limit the present invention nor impose any restrictions on it. Now, in the following Examples, as feeder cells, a human cell line grown on a medium comprising fetal bovine serum (FBS) was used. That is due to our situation wherein human cells obtained directly from a human body were hardly available for an experiment material, which forces us to obtain and use an existing human cell line for research purpose in these experiments.

### (Example 1)

A normal human diploid lung fibroblast cell line (MRC-5) obtained from Cell Bank and cultured into 41. 77 PDL (population doubling Level (The Japanese Tissue Culture Association Ed. "Tissue Culture Technology", 3rd ed. (basic) 1996, p42)) in a medium of 10 v/v% FBS in MEM was suspended at a cell count of 2.4 x 10⁶ cells using the following culture medium for preparation of feeder cells (A), and then cultured into 42.99 PDL on a gelatin-coated dish through one-passage of subculture.

### Composition of a culture medium for preparation of feeder cells (A):

RITC80-7 medium added with 5 g/L bovine serum albumin (BSA), 10 µg/L EGF, 1 mg/L insulin and 1 mg/L hydrocortisone

Then, the cultured MRC-5 described above was cultured for two to three hours using a culture medium for preparation of feeder cells (A) containing mitomycin C (MMC) at a final concentration of 10 µg/mL, followed by the inactivation of cell division. Then, the culture medium for preparation of feeder cells (A) containing MMC was removed and the cells were washed three times with phosphate buffer (PBS). The cells after washing were dissociated from the culture dish by trypsinization (0.25 w/v% trypsin, 1 mM EDTA) to count. As a result, approximately 5.5 x 10⁶ of feeder cells were obtained.

### (Example 2)

MRC-5 obtained from Cell Bank as in Example 1 was suspended at a cell count of 2.1 x 10⁵ cells using the following culture medium for preparation of feeder cells (B), and the cells were subcultured four passages into 53.47 PDL on a gelatin-coated culture dish.
Then, the cultured MRC-5 described above were cultured for two to three hours using a culture medium for preparation of feeder cells (B) containing MMC at a final concentration of 10 µg/mL, followed by the inactivation of cell division. Then, the culture medium for preparation of feeder cells (B) containing MMC was removed, and the cells were washed three times with PBS to produce feeder cells. These feeder cells were plated onto a gelatin-coated dish having a diameter of 60 mm at approximately 4 x 10⁵ per dish as the number of living cells and left to adhere.

### Composition of a culture medium for preparation of feeder cells (B):

A mixed culture medium of DMEM and Ham F in 1 : 1 added with 20 v/v% serum replacement (Knock out Serum Replacement: from Invitrogen (patent document No.1)) containing 83 g/L albumin and 100 mg/L insulin, 1 v/v% solution of MEM non-essential amino acid (from Invitrogen), 1mmol/L sodium pyruvate, 2mmol/L L-glutamine, 0.1 mmol/L 2-mercaptoethanol, 10 µg/L EGF and 1 µg/L FGF

After thawing a cryopreserved stock of embryonic stem cells from a cynomolgus monkey, a suspension of those cells at a concentration of approximately 2 x 10⁵ cells/mL as the number of living cells in the following culture medium for embryonic stem cells from a cynomolgus monkey (A) was plated onto the dish in which the feeder cells described above were left to adhere. The colonies of embryonic stem cells from a cynomolgus monkey were cultured in a 5% CO₂ incubator at 37°C, changing culture media for embryonic stem cells from a cynomolgus monkey (A) everyday until grown enough for subculture (for ten days).

### Composition of a culture medium for embryonic stem cells from a cynomolgus monkey (A)

A mixed culture medium of DMEM and Ham F in 1 : 1 added with 20 v/v% serum replacement (patent document No.1) containing 83 g/L albumin and 100 mg/L insulin, 1 v/v% MEM non-essential amino acid solution (from Invitrogen), 1mmol/L sodium pyruvate, 2mmol/L L-glutamine and 0.1 mmol/L 2-mercaptoethanol.

The colonies of cultured embryonic stem cells from a cynomolgus monkey described above were dissociated from the dish by trypsinization (0.25 w/v% trypsin). A part of the cells in the dish were plated again onto a dish containing new feeder cells, then culture was kept under the same conditions for four days, and the remaining cells in the dish were dissociated therefrom to count.

After finishing culture the subcultured colonies of embryonic stem cells, a part of the cells in the dish were dissociated from the dish similar to the above to count. The remaining cells in the dish were fixed with 4 w/v% paraformaldehyde, then immunostained with FITC-labeled SSEA-4 antibody (Santa Cruz Biotechnology, Inc), and observed with a 460-490 nm BP and a 515 nm BA by fluorescence microscopy.

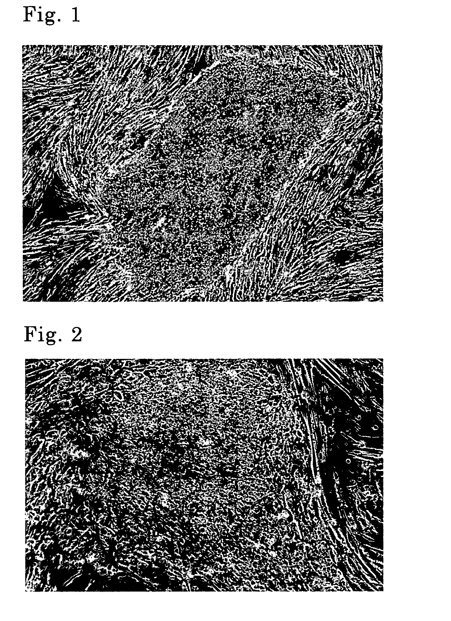

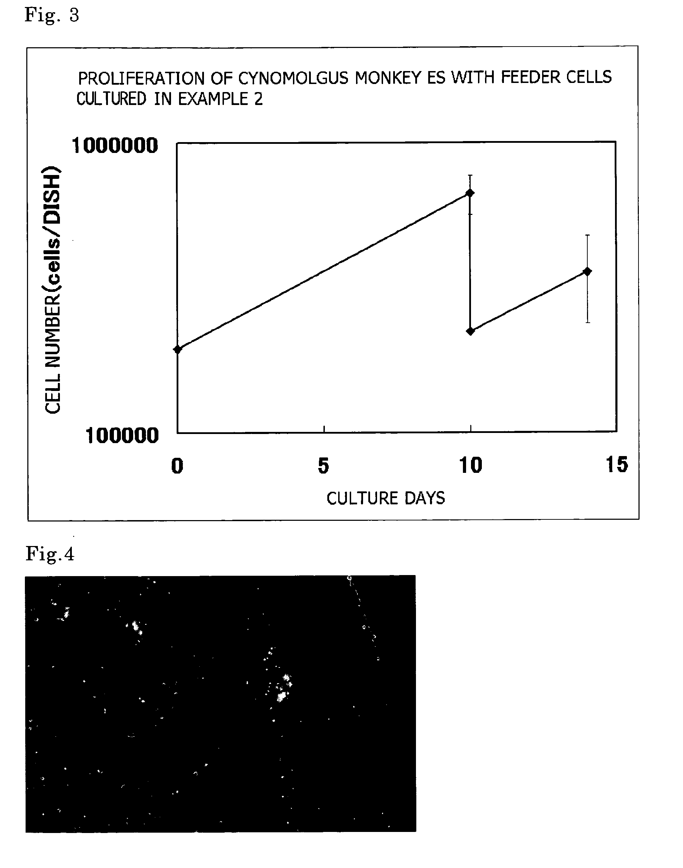

As a result, the embryonic stem cells from a cynomolgus monkey had an equivalent colony morphology as that cultured with the feeder cells derived from mouse fetal fibroblasts both during and after subculture (Figs. 1 and 2), showing the embryonic stem cells from a cynomolgus monkey were proliferated (Fig. 3). Further, in immunostaining at the end of culture, a fluorescence by SSEA-4, which is one of markers for embryonic stem cells from a primate, was observed (Fig. 4), proving it to be the colony of embryonic stem cells from a primate.
This result suggested that on feeder layer prepared from MRC-5 by the method according to the present invention, embryonic stem cells can be cultured.

### (Example 3)

MRC-5 obtained from Cell Bank as in Example 1 was suspended at a cell count of 2. 4 x 10⁶ cells using the culture medium for preparation of feeder cells (B) and subcultured four passages into 53.68 PDL on a collagen-coated culture dish. Then, the cultured MRC-5 described above was cultured for two to three hours in a culture medium for preparation of feeder cells (B) containing MMC at a final concentration of 10 µg/mL, followed by the inactivation of cell division. Then, the culture medium for preparation of feeder cells (B) containing MMC was removed and the cells were washed three times with PBS, preparation of feeder cells.

These feeder cells were plated onto a gelatin-coated dish having a diameter of 60 mm at approximately 4 x 10⁵ per dish as the number of living cells and left to adhere. After thawing a cryopreserved stock of embryonic stem cells from a cynomolgus monkey, a suspension of those cells at a concentration of approximately 2 x 10⁵ cells/mL as the number of living cells in the culture medium for embryonic stem cells from a cynomolgus monkey (A) was plated onto the dish in which the feeder cells were left to adhere. The colonies of embryonic stem cells were cultured in a 5% CO₂ incubator at 37°C, changing culture media everyday until grown enough for subculture (for ten days). The colonies of embryonic stem cells were dissociated by trypsinization (0.25 w/v% trypsin), and a part of the cells in the dish were plated again onto a dish containing new feeder cells, then culture was kept under the same conditions for four days, and the remaining cells in the dish were dissociated therefrom to count.

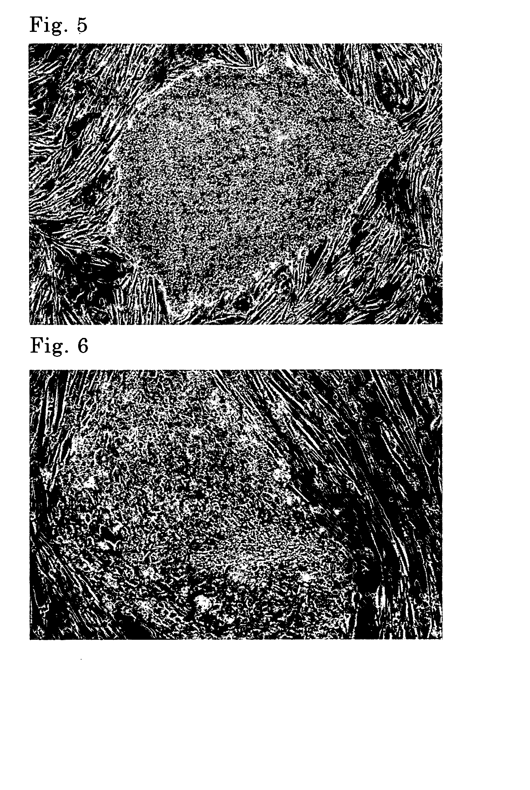

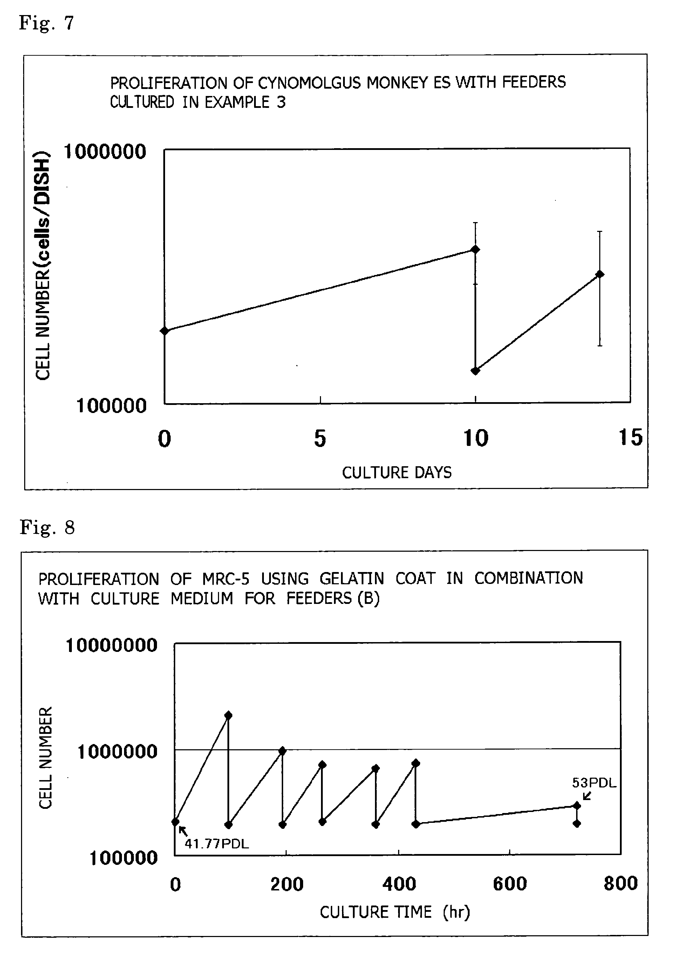

After finishing culture the subcultured colonies of embryonic stem cells, the cells were dissociated from the dish to count. As a result, the embryonic stem cells from a cynomolgus monkey had an equivalent colony morphology as that cultured with mouse feeder cells both during subculture and at the end of culture (Figs.5 and 6), showing the cells were proliferated (Fig. 7).
This result suggested that using a collagen-coated dish as in Example 2, embryonic stem cells can be cultured with the feeder cells prepared from MRC-5.

### (Example 4)

MRC-5 obtained from Cell Bank as in Example 1 was suspended at 2.1 x 10⁵ cells using a culture medium for preparation of feeder cells (B), then subculture was repeated on a gelatin-coated culture dish until the proliferation was declined, and the proliferation limit with a serum-free medium was examined. As a result, with a culture medium of the present invention, it was confirmed that fibroblasts available for feeder cells can be grown even from 41.77 PDL, subcultured up to four passages, and divided up to approximately 53 PDL (Fig. 8).
This result suggested that a substantial amount of feeder cells can be prepared from a single cell strain.

### (Example 5)

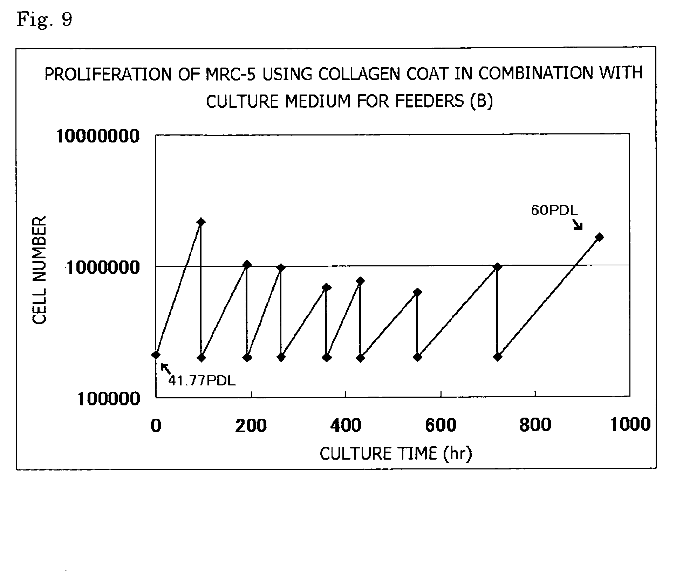

MRC-5 obtained from Cell Bank as in Example 1 was suspended at 2.1 x 10⁵ cells using a culture medium for preparation of feeder cells (B), then subculture was repeated on a collagen-coated culture dish until the proliferation was declined, and the proliferation limit with a serum-free medium was examined.
As a result, with a culture medium of the present invention, it was confirmed that fibroblasts available for feeder cells can be grown even from 41.77 PDL, subcultured up to seven passages, and divided into approximately 60 PDL, which is nearly the limit of the finite cell division of fibroblasts (Fig. 9).
This result suggested that a culture medium of the present invention could produce more feeder cells in combination with a collagen coat.

### (Example 6)

A normal human diploid lung fibroblast cell line (TIG-3) obtained from Cell Bank and cultured into 28.76 PDL in a medium of 10 v/v% FBS in MEM was suspended at 1.8 x 10⁵ cells using a culture medium for preparation of feeder cells (B) and then cultured into 35.51 PDL on a collagen-coated culture dish through two-passages of subculture.
Then, TIG-3 was cultured in a culture medium for preparation of feeder cells (B) containing MMC at a final concentration of 10 µg/mL for two to three hours, followed by the inactivation of cell division. Then, the culture medium containing MMC was removed and the cells were washed three times with PBS. The cells after washing were dissociated from the culture dish by trypsinization (0.25 w/v% trypsin, 1 mM EDTA) to count. As a result, approximately 2.1 x 10⁷ of feeder cells were obtained.
This result suggested that a large amount of feeder cells could be prepared from a fibroblast cell line other than MRC-5 by using a culture medium of the present invention.

### (Comparative Example 1)

MRC-5 obtained from Cell Bank as in Example 1 was suspended at 2.2 x 10⁵ cells using a culture medium for human primary fibroblasts which was a modified MCDB 202 free of serum albumin and added with 1 µg/L FGF and 5 mg/L insulin (hereinafter referred to as "FGM": from Cambrex) and cultured on a gelatin-coated culture dish through one-passage of subculture. However, the cell proliferation was inactivated after subculture, thereby the required MMC treatment was not conducted, resulting in difficulties in preparation of feeder cells.

### (Comparative Example 2)

MRC-5 obtained from Cell Bank as in Example 1 was suspended at 2.2 x 10⁵ cells using FGM and cultured on a collagen-coated culture dish through one-passage of subculture. However, similarly to Comparative Example 1, the cell proliferation was inactivated after subculture, thereby the required MMC treatment was not conducted, resulting in difficulties in preparation of feeder cells.

### Industrial Applicability

According to the present invention, feeder cells used in culture of embryonic stem cells including human's can be prepared with a serum-free medium with a reduced risk of zoonosis. Further, in combination with a cell adhesion factor such as gelatin and collagen, a long-term culture can be achieved, so that limited donor-derived materials can be used ultimately, resulting in considerably minimizing the risk of contamination of contagium which is caused by changing donors of feeder cells. In addition, as the present invention was confirmed to be available for a plurality of cell lines by Examples, it can be used in the preparation of respective feeder cells appropriate for embryonic stem cells derived from a variety of animals.

## Claims

1. A culture medium for preparation of feeder cells for embryonic stem cells, comprising serum albumin, insulin and a basal medium, wherein the amount of said serum albumin is from 2 g/L to 50 g/L, the amount of said insulin is from 1 mg/L to 100 mg/L, and said basal medium is at least one kind selected from MEM, α-MEM, DMEM, IMDM, Ham F10, Ham F12, Medium 199, RPMI 1640, RITC 80-7, MCDB 104, MCDB 105, MCDB 153, MCDB 201 and MCDB 202.

2. The culture medium according to claim 1 further comprising a cell adhesion factor.

3. The culture medium according to claim 2, wherein the cell adhesion factor is at least one kind selected from collagen, gelatin, fibronectin, vitronectin, laminin, polylysine, polyornithine and polyethyleneimine.

4. The culture medium according to claim 1, 2 or 3 further comprising a cell growth factor.

5. The culture medium according to claim 4, wherein the cell growth factor is at least one kind selected from fibroblast growth factor and epithelial cell growth factor.

6. A method for preparation of feeder cells for embryonic stem cells comprising the steps of:
culture and proliferation a cell population comprising at least one kind of cells selected from fetal skin fibroblasts, fetal myofibroblasts, fetal lung fibroblasts, fetal epithelial cells, fetal endothelial cells, adult skin fibroblasts, adult lung fibroblasts, adult epithelial cells and adult endothelial cells in the culture medium for preparation of feeder cells for embryonic stem cells according to any one of claims 1 through 5, and
inactivation of proliferation of said cultured and proliferated cell population by mitomycin C or irradiation.

7. The preparation method according to claim 6, wherein said culture and proliferation step is conducted in a culture vessel coated with a cell adhesion factor.

8. The preparation method according to claim 7, wherein the cell adhesion factor is at least one kind selected from collagen, gelatin, fibronectin, vitronectin, laminin, polylysine, polyornithine and polyethyleneimine.

9. The preparation method according to claim 6, 7 or 8, wherein in said culture and proliferation step, the cultured cells are allowed to undergo cell division twenty or more times on average.

10. Feeder cells for embryonic stem cells obtained by the preparation method according to any one of claims 6 through 9.
